# EUROPÄISCHE PATENTSCHRIFT

(11) **EP 2 313 072 B1**
(45) Veröffentlichungstag und Bekanntmachung des Hinweises auf die Patenterteilung: **20.08.2014**
(21) Anmeldenummer: 09780509.7
(22) Anmeldetag: 13.07.2009
(51) Int. Cl.: A61K 8/31, A61K 8/42, A61K 8/44, A61K 8/46, A61Q 5/02, A61Q 19/10, A61K 8/02, A61K 8/06

(54) **STRUKTURIERTE ZUSAMMENSETZUNG MIT OPTIMALEN LAGER-STABILITÄTS-EIGENSCHAFTEN**
STRUCTURED COMPOSITION WITH OPTIMUM STORAGE STABILITY PROPERTIES
COMPOSITION STRUCTURÉE À PROPRIÉTÉS DE STABILITÉ AU STOCKAGE OPTIMALES

(30) Priorität: 28.07.2008 DE 102008035172
(43) Veröffentlichungstag der Anmeldung: 27.04.2011
(73) Patentinhaber: Henkel AG & Co. KGaA, 40589 Düsseldorf (DE)
(72) Erfinder: HIPPE, Thomas, 25482 Appen (DE); BENDER, Corinna, 22457 Hamburg (DE); SCHRÖDER, Thomas, 22395 Hamburg (DE)
(86) Internationale Anmeldenummer: PCT/EP2009/058919
(87) Internationale Veröffentlichungsnummer: WO 2010/012582

(56) Entgegenhaltungen:
- WO-A1-03/055456
- WO-A2-2007/119226
- US-A1- 2001 053 753
- US-A1- 2006 128 579
- Sonneborn Refined Products: "Specifications for Super White Protopet® Petrolatum", Material Safety Data Sheet , 27. Juni 2008 (2008-06-27), XP002610613, Gefunden im Internet: URL:http://www.sonneborn.com/products/pdf/ Super_White_Protopet-PDS.pdf [gefunden am 2010-11-19]
- "International Cosmetic Ingredient Dictionary", 2006, The Cosmetic, Toiletry, and Fragrance Association, Washington DC, US ISBN: 1-882621-36-0 vol. 1, page 223, 418 und 419,

## Beschreibung

Die vorliegende Erfindung betrifft strukturierte Zusammensetzungen, die eine spezielle Kombination aus Tensiden und (einem) Ölkörper(n) enthalten.

Reinigungszusammensetzungen, die im kosmetischen Bereich eingesetzt werden, müssen heutzutage eine Vielzahl von Bedürfnissen abdecken, um marktfähig und für den Verbraucher attraktiv zu bleiben. Der Grund dafür besteht darin, dass beispielsweise ein Shampoo oder ein Duschgel nicht nur die Haut und/oder die Haare reinigen, sondern auch Haar- oder Hautschädigungen vorbeugen und mildem, Konditioniermittel auf der Anwendungsoberfläche abscheiden, und das äußere und innere Erscheinungsbild des Haares/der Haut verbessem soll. Zur Erfüllung all dieser Anforderungen wird eine Vielzahl kosmetischer Inhaltsstoffe in einer Formulierung benötigt, die aber häufig in negativer Weise miteinanderwechselwirken und sich damit desaktivieren können. Des Weiteren sind einige Komponenten, die erforderlich sind, nicht in der Matrix der Reinigungszusammensetzung löslich, was wiederum weitere Komponenten zur Suspendierung dieser Wirkstoffe notwendig macht.

Auch die Viskosität kosmetischer Reinigungszusammensetzungen spielt eine große Rolle, denn einige essentielle Wirkstoffe können nur bei besonders hohen Viskositätswerten stabil suspendiert werden, was anwendungstechnisch nicht wünschenswert ist, denn (beispielsweise) ein Shampoo muss leicht aus der Verpackung austreten, ohne zu flüssig zu sein, und muss sich gründlich im Haar verteilen lassen, was bei einer hohen Viskosität erschwert ist.

Sowohl die Art der Pflegekomponenten, als auch die rheologischen Eigenschaften einer kosmetischen Reinigungszusammensetzung stehen folglich bei der Herstellung moderner Formulierungen im Fokus.

Üblicherweise enthalten kosmetische Reinigungszusammensetzungen ein Primärtensid, das in erster Linie der Reinigung der Haare oder der Haut dient. Diese sind üblicherweise anionischer Natur, wobei Alkylsulfate und/oder Alkylethersulfate überwiegend eingesetzt werden. Für Formulierungen, die besonders mild sein sollen, ist man heutzutage bestrebt, entweder milde anionische Tenside einzusetzen, oder den Formulierungen einen Schaum-Booster bzw. einen Schaum-Stabilisator als Sekundärtensid hinzuzufügen. Bei den Sekundärtensiden handelt es sich üblicherweise um amphotere/zwitterionische und/oder nichtionische Tenside.

In modernen 2in1-Reinigungsprodukten spielt jedoch nicht nur die Reinigung, sondern auch die Pflege eine große Rolle, so dass solche Produkte auch noch eine oder mehrere Pflegekomponenten enthalten. Diese sind häufig nicht in der wässrigen Basis gängiger Reinigungszusammensetzungen löslich, weshalb man einen Weg finden muss, sie stabil zu suspendieren.

Es sind im Stand der Technik viele Lösungen für das Problem der Suspendierung unlöslicher Komponenten in einer wässrigen Tensid-Basis vorgeschlagen worden, problematisch ist allerdings bis heute, dass die Suspendierung unlöslicher Komponenten bei längerer Lagerung oder bei Temperaturschwankungen äußerst unbefriedigend ist

Ein Grund dafür ist, dass die Suspendierung der Komponenten häufig an eine bestimmte Viskosität geknüpft ist, und dass die Viskosität über einen längeren Zeitraum oder unter dem Einfluss hoher oder niedriger Temperaturen nicht stabil bleibt.

Um diesem Problem auf den Grund zu gehen, wurde die Rheologie tensidhaltiger Zusammensetzungen in den letzten Jahren intensiv untersucht.

Das rheologische Verhalten aller Tensidsysteme ist abhängig von ihrer Mikrostruktur, beispielsweise von der Form und der Konzentration von Micellen oder anderen zusammenhängenden Strukturgebilden in Lösungen. Micellen sind nicht zwangsläufig sphärisch und können beispielsweise zylindrisch sein. Bei hohen Tensidkonzentrationen erhält man geordnetere Mikrostrukturen wie lamellare, hexagonale oder kubische Phasen. Die Mikrostruktur tensidischer Systeme kann sich auch bei der sogenannten kritischen micellaren Konzentration (CMC) ändern.

Die Rheologie tensidischer Systeme wird mit den Begriffen Newton'sches und nicht-Newton'sches strukturviskoses Verhalten beschrieben. Newton'sche Tensidsysteme sind unabhängig von der Scherrate, während nicht-Newton'sche Tensidsysteme eine Viskosität aufweisen, die abhängig ist von der Scherrate. So wird die Viskosität eines nicht-Newton'schen frei fließenden Tensidsystems kleiner, wenn die Scherrate erhöht wird. Dieses nicht-Newton'sche rheologische Verhalten erlaubt die Suspendierung unlöslicher und ungelöster Feststoffe, Flüssigkeiten und/oder Gase.

Im Stand der Technik sind mehrere Methoden vorgestellt worden, mit denen sich lamellare Phasensysteme herstellen lassen. Beispielsweise betreffen die EP 1 037 601, WO 2003/055456, EP 1 165 019 und die EP 1 465 584 flüssige Tensidzusammensetzungen für kosmetische Zwecke, die auf diversen Tensid-Mischungen basieren und lamellare Phasen bzw. nicht-Newton'sches rheologisches Verhalten aufweisen.

In dem Dokument WO 2013/0455456A1 werden strukturierte wässrige Zusammensetzungen beschrieben, die ein anionisches Tensid, ein Alkanolamid und einen Elektrolyten enthalten, und nach Durchlaufen mindestens eines Gefrier-Tau-Zyklus eine stabile Viskosität aufweisen.

Es sind somit Techniken und Methoden bekannt, anhand derer lamellare Phasen und Zusammensetzungen mit nicht-Newton'schem rheologischem Verhalten herstelibar sind.

Bisher problematisch ist aber, dass die Stabilität solcher Systeme nicht immer erhalten werden kann. Insbesondere bei kälteren Temperaturen (beispielsweise -45°C bis 7°C) können solche Zusammensetzungen ihre lamellare Stabilität verlieren, Man nimmt an, dass dies darauf zurückzuführen ist, dass bei kalten Temperaturen die Öltröpfchen weniger flexibel werden und die kugelförmige Struktur, die die lamellare Wechselwirkung kennzeichnet, zerfällt.

Üblicherweise macht sich dieses Problem in einem drastischen Viskositätsabfall bemerkbar, der die Zusammensetzungen unbrauchbar und unattraktiv macht.

Aufgabe der vorliegenden Erfindung war es daher eine Zusammensetzung herzustellen, die die Suspendierung von Ölkörpern erlaubt und lamellare Phasen enthält.

Eine weitere Aufgabe der Erfindung bestand darin eine Zusammensetzung herzustellen, in der Ölkomponenten bei einer geeigneten Viskosität stabil suspendiert werden können, und deren Anfangsviskosität auch nach mehreren Gefrier-Tau-Zyklen im Wesentlichen erhalten bleibt.

Eine dritte Aufgabe der Erfindung war es eine Zusammensetzung mit einem möglichst hohen Gehalt an stabil suspendierten Ölkörpern herzustellen, die sich als kosmetische Haut - und Haarreinigungs-, sowie - konditioniermittel eignet.

Optimalerweise soll ein weiterer Haar- oder Hautpflegeschritt (beispielsweise Eincrernen der Haut oder Spulen der Haare mit einer Pflegespülung oder einer Haarkur) durch die alleinige Anwendung der Zusammensetzung, aus der die Pflege-Ölkomponente(n) auf der Anwendungsoberfläche abgeschieden wird (werden), überflüssig werden. Dies würde für den Verbraucher eine Zeit- und Kostenersparnis bedeuten.

Es wurde nun gefunden, dass sich eine spezielle Kombination mindestens dreier Tenside aus unterschiedlichen Tensidgruppen in Verbindung mit (einem) speziellen Ölkörper(n) zur Lösung dieser Aufgabe hervorragend eignet

Die entsprechenden Zusammensetzungen ermöglichen nicht nur die stabile Suspendierung der Ölkomponente bei einer angestrebten Viskosität, sondern weisen darüber hinaus auch eine besondere Viskositätsstabilität auf.

Damit eignen sich die Zusammensetzungen in hervorragender Weise für die Verwendung als kosmetisches Mittel zur Reinigung und Pflege menschlicher Haut und menschlicher Haare.

Gegenstand der vorliegenden Anmeldung ist in einer ersten Ausführungsform eine strukturierte wässrige Zusammensetzung, enthaltend ein Tensidgemisch, das - bezogen auf das Gesamtgewicht der Zusammensetzung -
(a) 3 bis 20 Gew.-% mindestens eines anionischen Tensids, das ein lineares oder verzweigtes Alkylsulfat und/oder -sulfonat ist, welches alkoxyliert sein kann,
(b) 0,5 bis 10 Gew.-% mindestens eines amphoteren/zwitterionischen Tensids und
(c) 0,5 bis 10 Gew.-% mindestens eines Alkanolamids, welches eine lineare, verzweigte, gesättigte oder ungesättigte aliphatische C-Kette und die folgende Struktur aufweist, 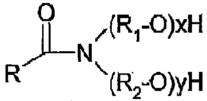 in der R eine C₈-C₂₄ gesättigte oder ungesättigte, lineare oder vernetzte aliphatische Gruppe, R₁ und R₂ gleich oder verschieden sind und eine C₂-C₄ lineare oder verzweigte aliphatische Gruppe bilden, x einen Wert von 0 bis 10 und y einen Wert von 1 bis 10 aufweist, wobei die Summe aus x+y kleiner oder gleich 10 ist, und
(d) einen Ölkörper mit einem Tropfpunkt >45°C, ausgewählt aus Petrolatum, einem Paraffin oder einem Silikon enthält,
dadurch gekennzeichnet, dass die Zusammensetzung weiterhin
(e) mindestens ein kationisches Tensid (e) der Formel 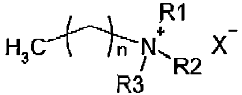 enthält, in der R1, R2 und R3 unabhängig voneinander ausgewählt sind aus -CH₃, - CH₂CH₃, -GH₂CH₂CH₃, -CH(CH₃)₂, n für einen ganzzahligen Wert von 9 bis 41 und X für ein Anion steht, und
dass die Zusammensetzung nach mindestens einem Gefrier-Tau-Zyklus eine Viskosität aufweist, die mindestens noch 80% der Anfangsviskosität beträgt.

In einer bevorzugten Ausführungsform der Erfindung weisen die Zusammensetzungen nach mindestens 3 Gefrier-Tau-Zyklen noch eine Viskosität von mindestens 80% der Anfangsviskosität auf.

Bei den erfindungsgemäß geeigneten Ölkörpern handelt es sich um alle für den Einsatz in tensidischen Zusammensetzungen geeigneten Ölkörper, die einen Tropfpunkt oberhalb von 45°C aufweisen. Der Tropfpunkt der Ölkomponente und damit die Wahl des Ölkörpers ist für die vorliegende Erfindung essentiell, denn es wurde festgestellt, dass Ölkomponenten mit einem niedrigeren Tropfpunkt in den erfindungsgemäßen Zusammensetzungen die vorgenannten Stabilitätsprobleme hinsichtlich der Suspendierung der Ölkomponente, der Ausbildung lamellarer Phasen und insbesondere der Erhaltung der Stabilität und Viskosität bei Temperaturschwankungen (insbesondere unter den drastischen Bedingungen eines Gefrier-Tauzyklus) aufwiesen.

Als Olkörper eignen sich Petrolatum, Paraffine und/oder Silikone, die einen Tropfpunkt oberhalb von 45°C aufweisen. Die Silikone werden an späterer Stelle dieser Erfindung näher beschrieben. Diese speziellen Ölkörper werden in den erfindungsgemäßen Zusammensetzungen - bezogen auf deren Gesamtgewicht- bevorzugt in einer Menge von 0,05 bis 15 Gew.-%, besonders bevorzugt in einer Menge von 0,1 bis 10 Gew.-% und insbesondere in einer Menge von 0,5 bis 5 Gew.-% eingesetzt.

Besonders geeignet sind Paraffine mit einem Tropfpunkt >45°C wie beispielsweise das unter dem Handelsnamen "Weichceresin FL 400" von der Firma PARAFLUID Mineralölgesellschaft GMBH vertriebene Petrolatum.

Eine essentielle Komponente der erfindungsgemäßen Tensidmischung ist ein anionisches Tensid, das ein lineares oder verzweigtes Alkylsulfat und/oder -sulfonat ist, welches alkoxyliert sein kann.

In einer bevorzugten Ausführung der Erfindung handelt es sich bei dem anionischen Tensid um ein lineares oder verzweigtes Alkylsulfat und/oder-sulfonat mit einer Alkylkettenlänge von 6 bis 20, bevorzugt von 8 bis 18 und insbesondere von 10 bis 16 C-Atomen mit einem Ethoxylierungsgrad von 0 bis 10, bevorzugt von 1 bis 8 und insbesondere von 2 bis 4. Besonders bevorzugte Beispiele geeigneter anionischer Tenside sind die Alkali- Ammonium- und/oder die Di- und Triethanolaminsalze 2- oder 3-fach ethoxylierter, verzweigter oder geradkettiger Lauryl-, Pareth- und/oder Tridecethsulfate wie Laureth(2)sulfate, Laureth(3)sulfate und Trideceth(2)sulfate und Trideceth(3)sulfate. Spezifische und insbesondere geeignete anionische Tenside sind beispielsweise Natriumlaurylethersulfat (2EO), Natriumlaurylethersulfat (3EO), Ammoniumlaurylethersulfat (2EO), Ammoniumlaurylethersulfat (3EO), Natriumlaurylsulfat, Ammoniumlaurylsulfat, Natriumtridecethsulfat (2EO), Natriumtrideceth-sulfat (3EO), Ammoniumtridecethsulfat (2EO) und Ammoniumtridecethsulfat (3EO).

Das oder die spezielle(n) anionsiche(n) Tensid(e) wird (werden) in den erfindungsgemäßen Zusammensetzungen - bezogen auf ihr Gesamtgewicht - bevorzugt in Mengen von 3 bis 20 Gew.-%, mehr bevorzugt von 4 bis 16 Gew.-% und insbesondere in Mengen von 5 bis 13 Gew.-% eingesetzt

Als geeignete zwitterionische Tenside werden solche oberflächenaktiven Verbindungen bezeichnet, die im Molekül mindestens eine quartäre Ammoniumgruppe und mindestens eine COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen In der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat. Unter ampholytischen Tensiden werden solche oberflächenaktiven Verbindungen verstanden, die außer einer C₈- C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobultersären, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂- C₁₀ - Acylsarcosin.

Besonders bevorzugte erfindungsgemäße Zusammensetzungen sind dadurch gekennzeichnet, dass sie amphotere(s) Tensid(e) aus den Gruppen der N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcoslne, 2-Alkylaminopropionsäuren mit jeweils etwa 8 bis 24 C-Atomen in der Alkylgruppe, Alkylaminoessigsäuren mit jeweils etwa 8 bis 24 C-Atomen in der Alkylgruppe, N-Kokosalkylaminopropionat, Kokosacylaminoethylaminopropionate, C₁₂- C₁₆ - Acylsarcosin, N-Alkyl-N,N-dimethylammonium-glycinate, beispielsweise Kokosalkyl-dimethylammoniumglycinat, N-Acyl-aminopropyl-N,N,dimethylammoniumglycinate, beispielsweise Kokosacylaminopropyl-dimethylammoniumglycinat, 2-Alkyl-3-carboxymethyl-3-hydroxyethyl-imidazoline mit jeweils 8 bis 18 C-Atomen in der Alkyl- oder Acylgruppe, Kokosacylaminoethylhydroxyethylcarboxymethylglycinat, der unter der INCI-Bezeichnung Cocamidopropyl Betain bekannten Verbindungen, der unter der INCl-Bezeichnung Disodium Cocoamphodiacetate bekannten Verbindungen enthalten, wobei erfindungsgemäß bevorzugte Zusammensetzungen das bzw. die amphotere(n) Tensid(e) in Mengen von 0,5 bis 10 Gew.-%, vorzugsweise von 0,75 bis 8 Gew.-% und insbesondere von 1 bis 7,5 Gew.-%, jeweils bezogen auf die gesamte Zusammensetzung, enthalten.

Als drittes Tensid enthalten die erfindungsgemäßen Mittel 0,5 bis 10 Gew.% mindestens eines Alkanolamids, das eine lineare, verzweigte, gesättigte oder ungesättigte aliphatische C-Kette enthält und die folgende Struktur aufweist: 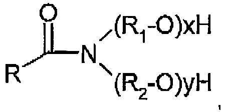 in der R eine C₈-C₂₄ gesättigte oder ungesättigte, lineare oder vernetzte aliphatische Gruppe, R₁ und R₂ gleich oder verschieden sind und eine C₂-C₄ lineare oder verzweigte aliphatische Gruppe bilden, x einen Wert von 0 bis 10 und y einen Wert von 1 bis 10 aufweist, wobei die Summe aus x+y kleiner oder gleich 10 ist.

Das oder die Alkanolamid(e) wird (werden) in den erfindungsgemäßen Zusammensetzungen - bezogen auf ihr Gesamtgewicht - bevorzugt in Mengen von 0,6 bis 8 Gew.-% und insbesondere in Mengen von 0,75 bis 6 Gew.-% eingesetzt

Spezifische Beispiele für geeignete Alkanolamide sind beispielsweise die unter den INCl-Bezeichnungen "Cocamide MEA", "Cocamide DEA" und "Cocamide MIPA" bekannten Verbindungen.

Erfindungsgemäß geeignete kationische Tenside weisen die folgende Formel (I) auf 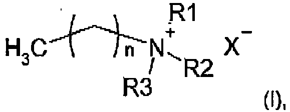 in der R1, R2 und R3 unabhängig voneinander ausgewählt sind aus -CH₃, - CH₂CH₃, -CH₂CH₂CH₃, -CH(CH₃)₂, n für einen ganzzahligen Wert von 9 bis 41 und X für ein Anion steht.

In ganz besonders bevorzugten Vertretern der Formel (I) gilt R1 = R2 = R3, so daß bevorzugte Zusammensetzungen dadurch gekennzeichnet sind, daß die Reste R1, R2 und R3 identisch sind. Unter den Resten R1, R2 und R3 sind Methylgruppen bevorzugt.

Die Anzahl der Methylengruppen im langkettigen Alkylrest kann erfindungsgemäß von 9 bis 41 variieren. Bevorzugt sind Verbindungen der Formel (I) mit langkettigen Alkylresten mit Werten für n von 9 bis 31, vorzugsweise von 11 bis 29, besonders bevorzugt von 13 bis 27, weiter bevorzugt von 15 bis 27 und insbesondere für 15, 16, 17, 18, 19, 20, 21.

In Formel (I) steht X für ein Anion, beispielsweise Fluorid, Chlorid, Bromid, Iodid, Hydrogensulfat, Sulfat, Dihydrogenphosphat, Hydrogenphosphat, Phosphat, p-Tuluolsulfonat usw. In bevorzugten Verbindungen steht X- für ein Halogenidanion, insbesondere für Chlorid.

Ganz besonders bevorzugte erfindungsgemäße Zusammensetzungen enthalten Cetrimoniumchlorid und/oder Behenyltrimethylammoniumchlorid.

Die kationischen Tenside werden in den erfindungsgemäßen Zusammensetzungen - bezogen auf ihr Gesamtgewicht - vorzugsweise in einer Menge von 0,01 bis 12,5 Gew.-% eingesetzt, wobei engere Mengenbereiche von 0,05 bis 10 Gew.-%, bevorzugte 0,1 bis 7,5 Gew.-% und insbesondere 0,25 bis 5 Gew.-% bevorzugt sind.

Neben den vorgenannten essentiellen Komponenten der erfindungsgemäßen Zusammensetzungen können diese noch weitere fakultative, bevorzugte Komponenten enthalten. So enthalten die Zusammensetzungen zur Unterstützung der erfindungsgemäßen Eigenschaften bevorzugt mindestens einen Elektrolyten.

Geeignete Elektrolyten können ausgewählt sein aus anorganischen und organischen Anionen, wie beispielsweise Halogenid-, Sulfat-, Phosphat-, Carbonat-, Citrat-, Benzoat- und/oder Tartrationen, und Alkali-, Ammonium- und/oder Erdalakli-Kationen wie Natrium-, Kalium-, Ammonium- und Magnesium-Kationen. Besonders bevorzugte Salze sind NaCl, Na₂SO₄, Nacitrat, Ammoniumchlorid, Ammoniumsulfat, MgCl₂, MgSO₄ und CaCl₂.

Die Salze können der Zusammensetzung sowohl direkt zugegeben werden, als auch als Teil eines Handelsprodukts. Ihr Gehalt in den erfindungsgemäßen Zusammensetzungen beträgt - bezogen auf das Gesamtgewicht der Zusammensetzung - bevorzugt 0,05 bis 10 Gew.-%, besonders bevorzugt 0,1 bis 8 Gew.% und insbesondere 0,5 bis 5 Gew.-%.

Neben den vorgenannten essentiellen Bestandteilen und den bevorzugten fakultativen Bestandteilen können die erfindungsgemäßen Zusammensetzungen weitere "benefit agents" enthalten. Das Vorliegen mindestens eines "benefit agents" in den erfindungsgemäßen Zusammensetzungen ist bevorzugt.

Unter "benefit agents" werden Wirkstoffe der folgenden Stoffgruppen verstanden: Antischuppenmittel, Konservierungsmittel, UV-Filter, pflanzliche Öle, Verdickungsmittel, Polymere, Feuchthaltemittel, Vitamin- und/oder Provitamine, Haarstrukturantien, Haarpflegemittel, Haarwuchsmittel und Mittel gegen Haarausfall.

Erfindungsgemäß geeignete Konservierungsmittel sind ausgewählt aus der Gruppe, die gebildet wird aus Benzoesäure, Propionsäure, Salicylsäure und/oder Sorbinsäure, sowie aus den kosmetisch verträglichen Salzen und Estern dieser Säuren, aus Formaldehyl und/oder Paraformaldehyd, aus Benzylalkohol, Germall, Dowicil, Kathon, Baypival, Methylisothiazoline, quaternären Ammoniumverbindungen, Alkoholen und/oder aus etherischen Ölen wie Teebaumöl, Cineol, Eugenol, Geraniol, Limonen, Menthol und/oder Thymol.

Sie werden den erfindungsgemäßen Zusammensetzungen - bezogen auf ihr Gesamtgewicht - in einer Menge von 0,01 bis 5 Gew.-%, bevorzugt in einer Menge von 0,05 bis 3 Gew.% und insbesondere in einer Menge von 0,1 bis 2 Gew.% hinzugefügt.

Erfindungsgemäß ganz besonders bevorzugte Konservierungsmittel sind Benzoesäure, Salicylsäure und/oder ein kosmetisch verträgliches Salz oder ein kosmetisch verträglicher Ester dieser Säuren, Benzylalkohol, Dowicil, Kathon, Baypival, Methylisothiazoline sowie Gemische dieser Stoffe.

Geeignete Antischuppenmittel werden den erfindungsgemäßen Zusammensetzungen in einer Menge von 0,05 bis 5 Gew.-%, bevorzugt von 0,1 bis 3,0 Gew.% und insbesondere von 0,3 bis 2,0 Gew.-% zugegeben (bezogen auf das gesamte Mittel) und sind ausgewählt aus Piroctone Olamine, Climbazol, Zink Pyrithion, Ketoconazole, Salicylsäure, Schwefel, Selensulfide, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten.

Erfindungsgemäß bevorzugt sind Salicylsäure, Climbazol, Zink Pyrithion und Piroctone Olamine. Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemaß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern. Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalol^{®} HP 610).

Die UV-Filter (I) sind in den erfindungsgemäßen Mitteln üblicherweise in Mengen 0,1-5 Gew.%, bezogen auf das gesamte Mittel, enthalten. Mengen von 0,4-2,5 Gew.% sind bevorzugt.

In einer weiteren bevorzugten Ausführungsform der Erfindung kann die Wirkung des erfindungsgemäßen Mittels durch den Zusatz pflanzlicher Ole optimiert werden.

Als natürliche Öle kommen beispielsweise Amaranthsamenöl, Aprikosenkernöl, Arganöl, Avocadoöl, Babassuöl, Baumwollsaatöl, Borretschsamenöl, Camalinaöl, Distelöl, Erdnußöl, Granatapfelkemol, Grapefruitsamenöl, Hanföl, Hagebuttenkernöl, Haselnussöl, Holundersamenöl, Johannisbeersamenöl, Jojobaöl, Kakaobutter, Leinöl, Macadamianussöl, Maiskeimöl, Mandelöl, Marulaöl, Nachtkerzenöl, Olivenöl, Palmöl, Pfirichkernöl, Rapsöl Reisöl, Sanddornfruchtfleischöl, Sanddornkernöl, Sesamöl, Sheabutter, Sojaöl, Sonnenblumenöl, Traubenkernöl, Walnußöl oder Wildrosenöl in Frage.

Bevorzugte natürliche Öle enthalten mindestens Palmitinsäure, Stearinsäure und Linolsäure. Besonders bevorzugte natürliche Öle enthalten die Fettsäuren Palmitinsäure, Stearinsäure und Linolsäure in einer Gesamtmenge von mindestens 50 Gew.% der Fettsäuren,

Erfindungsgemäß besonders bevorzugte natürliche Öle sind Aprikosenkemöl. Hagebuttenkernöl, Jojobaöl, Mandelöl, Olivenöl, Arganöl, Amaranthsamenöl, Sheabutter und Kakaobutter.

Die pflanzlichen Öle werden in den erfindungsgemäßen Mitteln - bezogen auf das Gesamtgewicht der Mittel in einer Menge von 0,001 bis 10 Gew-%, bevorzugt von 0,005 bis 7 Gew.-%, besonders bevorzugt von 0,01 bis 5 Gew.% und insbesondere von 0,05 bis 3 Gew.% eingesetzt.

Als weiterhin vorteilhaft hat es sich gezeigt, wenn Polymere in den erfindungsgemäßen Mitteln enthalten sind. In einer bevorzugten Ausführungsform werden den erfindungsgemäßen Mitteln daher Polymere zugesetzt, wobei sich sowohl kationische, anionische, amphotere als auch nichtionische Polymere als wirksam erwiesen haben.

Polymere haben in den erfindungsgemäßen Zusammensetzungen mehrere Fuktionen. Zum einen dienen insbesondere kationische Polymere in kosmetischen Zusmamensetzungen (und die erfindungsgemäßen Zusammensetzungen können als kosmetische Zusammensetzung dienen) als Konditioniermittel für die Haut und die Haare. Kationische Polymere dienen aber auch als Abscheidungshilfsmittel für Fette, Wachse und Öle, sowie für Silikone.

Weiterhin haben Polymere filmbildende Eigenschaften und häufig verdickende Eigenschaften, weshalb ihr Einsatz in Zusammensetzungen, in denen die Viskositätsstabilität eine große Rolle spielt, ebenfalls bevorzugt ist.

Besonders bevorzugt ist der Einsatz kationischer Polymere, die den erfindungsgemäßen Zusammensetzungen - bezogen auf ihr Gesamtgewicht - in einer Menge von 0,01 bis 5 Gew.-%, bevorzugt von 0,02 bis 3 und insbesondere von 0,05 bis 2 Gew.-%, zugegeben werden.

Unter geeigneten, kationischen Polymeren sind Polymere zu verstehen, welche in der Haupt- und/oder Seitenkette Gruppen aufweisen, welche "temporar" oder "permanent" kationisch sein können. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten, aufgebaute Polymerhauptkette gebunden sind, haben sich als besonders geeignet erwiesen. Homopolymere der allgemeinen Formel (PI), 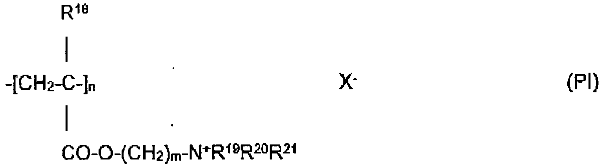 in der R¹⁶= -H oder-CH₃ ist, R¹⁹, R²⁰ und R²¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X-ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁸ steht für eine Methylgruppe
- R¹⁹, R²⁰ und R²¹ stehen für Methylgruppen
- m hat den Wert 2,

Als physiologisch verträgliches Gegenionen X- kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid)(INCl-Bezeichnung Polyquaternium-37). Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95, Tridecyl-polyoxypropylen-polyoxyethylen-ether (INCI-Bezeichnung: PPG-1-Trideceth-6)) und Salcare^{®} SC 96 (INCI-Bezeichnung: Propylene Glycol Dicaprylate/Dicaprate) sowie Tridecyl-polyoxypropylen-polyoxyethylen-ether (INCl-Bezeichnung: PPG-1-Trideceth-6)) im Handel erhältlich.

Copolymere mit Monomereinheiten gemäß Formel (PI) enthalten als nichtionogene Monomereinheiten bevorzugt Acrylamid, Methacrylamid, Acrylsäure-C₁₋₄-alkylester und Methacrylsaure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich. Weitere, besonders bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- hydrophob modifizierte kationische Polymere, wie sie beispielsweise unter der Handelbezeichnung SoftCat^{®} und Polymer^{®} SL im Handel erhältlich sind
- kationische Alkylpolyglycoside,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; di-quaternäre Polydimethylsiloxane, Quaternium-80),
- polymere Dimethyldiallylammoniumsalze und deren Copolymere mit Estern und Amiden von Acrylsaure und Methacrylsaure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte VinylpyrrolidonDimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quaternierter Polyvinylalkohol sowie die unter den Bezeichnungen
- Polyquaternium 2, Polyquaternium 17, Polyquaternium 18 und Polyquaternium 27 bekannten

Polymeren mit quartaren Stickstoffatomen in der Polymerhauptkette. Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind. Erfindungsgemäß insbesondere bevorzugte kationische Polymere sind die unter den Bezeichnungen Celquat^{®} und Polymer JR^{®} im Handel erhältlichen quaternierten Cellulosederivate (Polyquaternium-10 und Polyquaternium-4) sowie kationische Guar-Derivate, wie insbesondere die unter den Handelsnamen Cosmedia^{®}Guar und Jaguar^{®} vertriebenen Produkte. Entsprechende Handelsprodukte sind u.a. erhältlich unter den Bezeichnungen Polymer^{®} JR 400, Celquat^{®} H 100, Celquat^{®} L 200, Jaguar^{®} Excel und Jaguar^{®} C17.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein. Ganz besonders bevorzugt sind die kationischen Proteinhydrolysate und -derivate auf pflanzlicher Basis. Sie gelten auch als "Haarstrukturant" und werden daher bevorzugt in den erfindungsgemäßen Zusammensetzungen in Mengen von 0,001 bis 10 Gew.-%, mehr bevorzugt in Mengen von 0,005 bis 5 Gew.-% und insbesondere in Mengen von 0,01 bis 3 Gew.-% eingesetzt (bezogen auf das Gesamtgewicht der Mittel).

Weiterhin sind die Provitamine Panthenol und Pantolacton, das Vitaminderivat Niacinamid sowie die Wirkstoffe Arginin, Acetyltyrosin und Sericin zu den "Haarstrukturantien" zu zählen. Ihr Einsatz in in erfindungsgemäßen Zusammensetzungen, die als kosmetisches Haarbehandlungsmittel konfektioniert werden, ebenfalls bevorzugt. Die einzelnen Stoffe werden an späterer Stelle der Anmeldung näher beschrieben.

Zusätzlich kann es sich als vorteilhaft erweisen, in erfindungsgemäßen Zusammensetzungen Feuchthaltemittel bzw. Penetrationshilfsstoffe und/ oder Quellmittel (M) einzusetzen. Diese Hilfsstoffe können eine bessere Penetration von Wirkstoffen in die keratinische Faser bewirken oder helfen, die keratinische Faser aufzuquellen. Hierzu sind beispielsweise zu zählen Harnstoff und Harnstoffderivate, Guanidin und dessen Derivate, Arginin und dessen Derivate, Wasserglas, Milchsäure, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiof, 1,3-Propandiol, 1,6-Hexandiol, 1,5-Pentandiol, 1,4-Butandiol.

Erfindungsgemäß besonders geeignet sind Milchsäure, Glycerin und/oder Arginin.

Die Feuchthaltemittel werden in den erfindungsgemäßen Zusammensetzungen - bezogen auf die gesamte Zusammensetzung - in Mengen von 0,01 bis 10 Gew.-%, bevorzugt in Mengen von 0,05 bis 5 Gew.-% und insbesondere in Mengen von 0,1 bis 3 Gew.% eingesetzt.

Eine weitere bevorzugte Gruppe von Inhaltsstoffen in den erfindungsgemäßen Zusammensetzungen sind Vitamine, Provitamine oder Vitaminvorstufen.

Zur Gruppe der als Vitamin A bezeichneten Substanzen gehören das Retinol (Vitamin A1) sowie das 3,4-Didehydroretinol (Vitamin A2). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05 - 1 Gew.%, bezogen auf die gesamte Zubereitung.

Neben den in den erfindungsgemäßen Zusammensetzungen besonders bevorzugt einzusetztenden Pantolacton und Pantothensäure und/oder ihren Derivaten gehören zu erfindungsgemäß einsetzbaren Wirkstoffen der Vitamin B-Gruppe Vitamin B₁ (Thiamin), Vitamin B (Riboflavin), Vitamin B₃ (unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist), Vitamin B_{B} (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und H. Panthenol und seine Derivate sowie Nicotinsäureamid und Biotin sind besonders bevorzugt.

Die erfindungsgemäßen Mittel können zusätzlich zu den vorstehend genannten Inhaltsstoffen und optionalen weiteren Inhaltsstoffen weitere Stoffe enthalten, die Haarausfall verhindern, lindern oder heilen. Insbesondere ist ein Gehalt an haarwurzelstabilisierenden Wirkstoffen vorteilhaft. Diese Stoffe werden nachstehend beschrieben:
Propecia (Finasterid) ist das zur Zeit einzige Präparat, das weltweit zugelassen ist und für das in zahlreichen Studien eine Wirksamkeit und Verträglichkeit nachgewiesen wurde. Propecia bewirkt, dass sich weniger DHT aus Testosteron bilden kann. Minoxidil ist mit oder ohne ergänzende Zusatzstoffe das wohl älteste nachweislich wirkende Haarwuchsmittel. Zur Behandlung von Haarausfall darf es nur zur äußeren Anwendung verwendet werden. Es gibt Haarwasser, die 2%-5% Minoxidil enthalten, außerdem Gels mit bis zu 15% Minoxidil. Die Wirksamkeit nimmt mit der Dosierung zu, in Haarwassern ist Minoxidil jedoch nur bis zu 5% Anteil löslich. In vielen Ländern sind Haarwasser mit bis zu 2% Minoxidilgehalt verschreibungsfrei erhältlich.

Zusammenfassend sind erfindungsgemäße Haarbehandlungsmittel bevorzugt, die zusätzlich - bezogen auf sein Gewicht - 0,001 bis 5 Gew.-% Haarwurzel-stabilisierende Stoffe, insbesondere Minoxidil und/oder Finasterid und/oder Ketoconazol enthalten.

Als weiteren Inhaltsstoff können die erfindungsgemäßen Mittel mit besonderem Vorzug eine oder mehrere Aminosäuren bzw. deren physiologisch verträgliche Derivate enthalten, da diese Stoffe auch als "Haarstrukturantien" dienen, und deren Einsatz damit in erfindungsgemäßen Zusammensetzungen, die als kosmetisches Haarbehendlungsmittel konfektioniert werden, äußerst bevorzugt ist. Erfindungsgemäß besonders bevorzugt einsetzbare Aminosäuren bzw. Aminosäurederivate stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Camitin, L-Citrullin, L-Theanin, 3',4'-Dihydroxy-L-phenylalanin (L-Dopa), 5'-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren und/oder deren Derivate als auch deren Mischungen eingesetzt werden können.

Bevorzugte erfindungsgemäße Mittel enthalten eine oder mehrere Aminosäuren bzw. deren Derivate in engeren Mengenbereichen. Hier sind erfingdungsgemäß bevorzugte Zusammensetzungen dadurch gekennzeichnet, dass sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n)/-derivate, vorzugsweise aus der Gruppe Glycin und/oder Alanain und/oder Valin und/oder Lysin und/oder Leucin und/oder Threonin und/oder Arginin und/oder Acetyltyrosin enthalten.

Eine weitere Gruppe bevorzugter Komponenten in den erfindungsgemäßen Mitteln für den Fall, dass sie als kosmetisches Haarbehandlungsmittel formuliert werden, sind die Haarpflegemittel. Zu ihnen zählen die bereits vorgenannten pflanzlichen Öle, Panthenol, Hydrogenated Jojoba Oil, Biotin und Allantoin, aber auch die Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertigeAlkoholewie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - so Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Erfindungsgemäß besonders bevorzugte Mittel sind dadurch gekennzeichnet, dass sie - bezogen auf ihr Gewicht - 0,001 bis 10 Gew.-%, insbesondere 0,01 bis 2 Gew.-% Wirkstoff (=Pflanzenextrakt), enthalten.

Zusätzlich zu allen vorgenannten Inhaltsstoffen kann als eine besonders bevorzugte Komponente aller erfindungsgemäßen Zusammensetzungen ein Silikon dienen.

Auch Silikone können in den erfindungsgemäßen Mitteln verschiedene Funktionen erfüllen. So können sie zum einen als Strukturant dienen und - sofern sie einen Tropfpunkt oberhalb von 45°C aufweisen - alternativ zu den Paraffinen eingesetzt werden.

Sie können aber auch als Haut- und Haarpflegemittel dienen - ihr Einsatz in den erfindungsgemäßen Zusammensetzungen ist damit ebenfalls besonders bevorzugt.

Silikone werden in den erfindungsgemäßen Zusammensetzungen in Mengen von 0,1 bis 10 Gew.-%, vorzugsweise von 0,25 bis 5 Gew.-% und insbesondere von 0,5 bis 2,5 Gew.-%, jeweils bezogen auf die gesamte Zusammensetzung, eingesetzt.

Insbesondere bevorzugt sind erfindungsgemäße Mittel, die mindestens ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter substituierten oder unsubstituierten aminierten Gruppen; (per)fluorierten Gruppen; Thiolgruppen, Carboxylatgruppen; hydroxylierten Gruppen; alkoxylierten Gruppen; Acyloxyalkylgruppen; amphoteren Gruppen; Bisulfitgruppen; Hydroxyacylaminogruppen; Carboxygruppen; Sulfonsäuregruppen; und Sulfat- oder Thiosulfatgruppen;
(iii) linearen Polysiloxan(A)- Polyoxyalkylen(B)- Blockcopoylmeren vom Typ (A-B)ₙ mit n>3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Zusätzlich zu den vorgenannten, zwingend enthaltenen Tensiden können die erfindungsgemäßen Mittel auch weitere anionische, gegebenenfalls auch weitere amphotere bzw. zwitterionische Tenside sowie nichtionische Tenside enthalten.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alpha-Olefinsulfonate mit 8 bis 24 C-Atomen,
- Alpha-Sulfofettsäuremethylester von Fettsäuren mit 8 bis 30 C-Atomen,
- Gemische oberflächenaktiver Hydroxysulfonate,
- sulfatierte hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 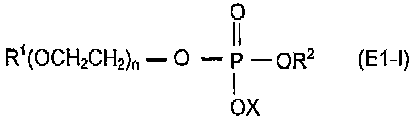 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1 -II)

   R⁷CO(AlkO)ₙSOsM (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Monoglyceridsulfate und Monoglyceridethersulfate der Formel (E1-III) 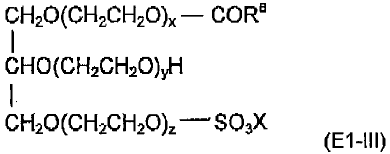 in der R^{a}CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (EI-III) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Nichtionische Tenside und Emulgatoren enthalten als hydrophile Gruppe z. B. eine Polyolgruppe, eine Polyalkylenglycolethergruppe oder eine Kombination aus Polyol- und Polyglycolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diester von Anlagerungsprodukten von 1 bis 30 Mol Ethylenoxid an Polyole mit 3 bis 6 Kohlenstoffatomen, insbesondere an Glycerin,
- Anlagerungsprodukte von 5 bis 60 Mol Ethylenoxid an Rizinusöl und gehärtetes Rizinusöl,
- Polyolfettsäure(partial)ester, wie Hydagen^{®} HSP (Cognis) oder Sovermol^{®} - Typen (Cognis), insbesondere von gesättigten C₈₋₃₀-Fettsäuren,
- alkoxylierte Triglyceride,
- alkoxylierte Fettsäurealkylester,
- Aminoxide,
- Fettsäurealkanolamide, Fettsäure-N-alkylglucamide und Fettamine sowie deren Ethylenoxid- oder Polyglycerin-Anlagerungsprodukte,
- Sorbitanfettsäureester und Anlagerungsprodukte von Ethylenoxid an Sorbitanfettsäureester wie beispielsweise die Polysorbate,
- Zuckerfettsaureester und Methylglucosid-Fettsäureester sowie deren Ethylenoxid- oder Polyglycerin-Anlagerungsprodukte,

Besonders bevorzugte nichtionische Tenside sind Alkylpolyglycoside. Demnach sind erfindungsgemäße Haarreinigungsmittel bevorzugt, die als nichtionische Tenside Alkylpolyglycoside der allgemeinen Formel RO-(Z)x enthalten, wobei R für Alkyl, Z für Zucker sowie x für die Anzahl der Zuckereinheiten steht.

Erfndungsgemäß bevorzugt werden Alkylpolyglycoside entsprechend der allgemeinen Formel RO-(Z)x eingesetzt, wobei R für Alkyl, Z für Zucker sowie X für die Anzahl der Zuckereinheiten steht.

Besonders bevorzugt sind solche Alkylpolyglycoside, bei denen R
- im wesentlichen aus C₈- und C₁₀-Alkylgruppen,
- im wesentlichen aus C₁₂- und C₁₄-Alkylgruppen,
- im wesentlichen aus Ce- bis C₁₆-Alkylgruppen oder
- im wesentlichen aus C₁₂- bis C₁₆-Alkylgruppen oder
- im wesentlichen aus C₁₆ bis C₁₈-Alkylgruppen
besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.

Weitere Pflegekomponenten, die insbesondere in erfindungsgemäßen Zusammensetzungen eingesetzt werden können, die als Haarbehandlungsmittel formuliert werden, sind bestimmte Chinone, die eine besondere Eignung als Pflege-Enhancer besitzen. Als weiteren Pflege-Enhancer können die erfindungsgemäßen Mittel daher 0,0001 bis 5 Gew.-% mindestens eines Biochinons der Formel (Ubi) 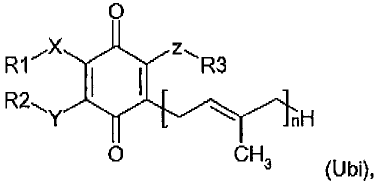 enthalten in der
- X, Y, Z: stehen unabhängig voneinander für -O- oder -NH- oder NR⁴- oder eine chemische Bindung
R¹/R²/R³ stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H, - CH₃, -CH₂CH₃, -(CH₂)₂CH₂, -CH(CH₃)₂, -(CH₂)₃CH₃, -CH(CH₃)CH₂CH₃, - CH₂CH(CH₃)₂, -C(CH₃)₃
R⁴ steht für -CH₃, -CH₂CH₃, -(CH₂)₂CH₂, -CH(CH₃)₂, -(CH₂)₃CH₃, -CH(CH₃)CH₂CH₃, - CH₂CH(CH₃)₂, -C(CH₃)₃
n steht für Werte von 1 bis 20, vorzugsweise von 2 bis 15 und insbesondere für 5, 6, 7, 8, 9, 10.

Besonders bevorzugte erfindungsgemäße Mittel sind dadurch gekennzeichnet, daß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 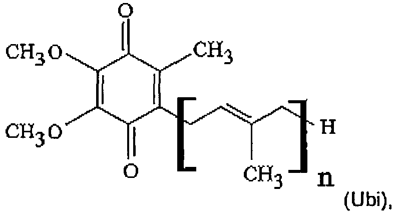 in der n für die Werte = 6, 7, 8, 9 oder 10, besonders bevorzugt für 10 (Coenzym Q10) steht.

Als weiteren Pflege-Enhacer können die erfindungsgemäßen Mittel Ectoin enthalten. Ectoin ((4S)-2-Methyl-1,4,5,6-Tetrahydropyrimldin-4-Carbonsäure) ist ein zur Gruppe der kompatiblen Solute gehörender Naturstoff. Die stark wasserbindende niedermolekulare organische Verbindung tritt in halophilen Bakterien auf und ermöglicht diesen extremophilen Organismen unter Stressbedingungen zu überleben. Erfindungsgemäß bevorzugte Haarreinigungsmittel sind dadurch gekennzeichnet, dass sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

Ein weiterer Pflege-Enhancer ist Allantoin. Allantoin (5-Ureidohydantoin, N-(2,5-Dioxo-4-imidazolidinyl)-harnstoff) wird in der Kosmetik in Hautcremes, Sonnenschutzmitteln, Rasierwässern, in Zahncreme und in Mitteln gegen übermäßige Schweißabsonderung (Hyperhidrose) und Hautirritationen eingesetzt. Es bewirkt die Beschleunigung des Zellaufbaus, der Zellbildung oder der Zellregeneration und beruhigt die Haut. Auch die Heilung schwer heilender Wunden wird unterstützt, jedoch besitzt Allantoin keine antiseptischen Eigenschaften.

Erfindungsgemäße besonders bevorzugte Haarreinigungsmittel enthalten - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% 5-Ureidohydantoin (Allantoin).

Als Pflege-Enhancer können die erfindungsgemäßen Mittel auch Flavonoide enthalten. Die Flavonoide sind eine Gruppe von wasserlöslichen Pflanzenfarbstoffen und spielen eine wichtige Rolle im Stoffwechsel vieler Pflanzen. Sie gehören zusammen mit den Phenolsäuren zu den Polyphenolen. Es sind weit über 6500 unterschiedliche Flavonoide bekannt, die sich in Flavonole, Flavone, Flavanone, Isoflavonoide und Anthocyane einteilen lassen.

Erfindungsgemäß können Flavonoide aus allen sechs Gruppen eingesetzt werden, wobei bestimmte Vertreter aus den einzelnen Gruppen als Pflege-Enhancer wegen ihrer besonders intensiven Wirkung bevorzugt sind. Bevorzugte Flavonole sind Quercetin, Rutin, Kaempferol, Myricetin, Isorhamnetin, bevorzugte Flavanole sind Catechin, Gallocatechin, Epicatechin, Epigallocatechingallat, Theaflavin, Thearubigin, bevorzugte Flavone sind Luteolin, Apigenin, Morin, bevorzugte Flavanone sind Hesperetin, Naringenin, Eriodictyol, bevorzugte Isoflavonoide sind Genistein, Daidzein, und bevorzugte Anthocyanidine (Anthocyane) sind Cyanidin, Delphinidin, Malvidin, Pelargonidin, Peonidin, Petunidin.

Erfindungsgemäß besonders bevorzugte Mittel sind dadurch gekennzeichnet, dass sie- bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-0-rutinosid (Rutin), enthalten.

Bevorzugt ist auch der Einsatz von Bisabolol und/oder Bisabololoxiden als Pflege-Enhancer in den erfindungsgemäßen Mitteln. Hier sind erfindungsgemäße Mittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol enthalten.

Auch Creatin eignet sich erfindungsgemäß als Pflege-Enhancer. Creatin (3-Methylguanidinoessigsäure) ist eine organische Saure, die in Wirbeltieren u. a. zur Versorgung der Muskeln mit Energie beiträgt. Kreatin wird in der Niere, der Leber und in der Bauchspeicheldrüse synthetisiert. Sie leitet sich formal von den Aminosäuren Glycin und Arginin ab und ist zu 95 % im Skelettmuskel vorhanden. Besonders bevorzugte erfindungsgemäße Mittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% N-Methyl-guanidino-essigsäure (Creatin).

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Mittel dadurch gekennzeichnet, dass sie als Pflegestoff - bezogen auf ihr Gewicht-0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus
- Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose
- Disacchariden, insbesondere Saccharose und/oder Maltose und/oder Lactose und/oder Trehalose und/oder Cellobiose und/oder Gentiobiose und/oder Isomaltose.

Die erfindungsgemäßen Mittel können weiterhin eine 2-Pyrrolidinon-5-carbonsäure und deren Derivate enthalten. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf das gesamte Mittel, besonders bevorzugt 0,1 bis 5, und insbesondere 0,1 bis 3 Gew.%.

In einer weiteren bevorzugten Ausführungsform können die erfindungsgemäßen Mittel Emulgatoren enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Pettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 C-atomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit B bis 30 C -Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Die erfindungsgemäßen Zusammensetzungen weisen üblicherweise einen pH-Wert im Bereich von 3 bis 8 auf. pH-Werte im Bereich von 3,5 bis 7,5, bevorzugt von 4 bis 7 und insbesondere von 4,5 bis 6,5 sind von besonderem Vorteil.

Ein zweiter Gegenstand der vorliegenden Erfindung ist die kosmetische Verwendung einer erfindungsgemäßen Zusammensetzung zur Reinigung und Pflege menschlicher Haut und menschlicher Haare.

Bezüglich bevorzugter Ausführungsformen der erfindungsgemäßen Verwendung gilt mutatis mutandis das zu den erfindungsgemäßen Mitteln Gesagte.

### Beispiele:

Die folgenden Beispiele erläutern die Erfindung.

Alle Angaben sind - sofern nicht anders angegeben - in Gewichtsprozent.

| **Rohstoff** | **1** | **2** | **3** | **4** | **5** |
|---|---|---|---|---|---|
| **Sodium Trideceth Sulfate** | 15 | 15 | | | |
| **Sodium Laureth Sulfate** | | | 15 | 15 | 15 |
| **Sodium Lauroamphoacetate** | 4,5 | | 4,5 | | |
| **Disodium Cocoamphodlacotate** | | | | 5 | |
| **Cocamidopropyl Betain** | | 5 | | | 4 |
| **Sodium Cloride** | 3 | 3 | 3 | 3 | 3 |
| **Cocamide MIPA** | 3,5 | | 3,5 | 3,5 | |
| **Cocamide MEA** | | 4 | | | 4 |
| **Petrolatum (Tropfpunkt >45°C)** | 4 | 4 | 4 | 4 | 4 |
| **Lactic Acid** | 0,5 | 0,5 | 0,5 | 0,5 | 0,5 |
| **Parfum** | 0,5 | 0,5 | 0,5 | 0,5 | 0,5 |
| **Dimethicone** | 1,2 | | 1,2 | 1,2 | 1,2 |
| **Guar Hydroxypropyl Trimonium Chloride** | 0,25 | | | | 0,25 |
| **Polyquaternium-10** | | 0,4 | 0,4 | 0,4 | 0,4 |
| **Behentrimonium Chloride** | 1 | 1 | 1 | 1 | 1 |
| **Coconut Alcohol** | 0,75 | 0,75 | 0,75 | 0,75 | 0,75 |
| **Methyllsothiazolinone** | 0,01 | 0,01 | 0,01 | 0,01 | 0,01 |
| **Wasser** | ad 100 | ad 100 | ad 100 | ad 100 | ad 100 |
| **pH-Wert** | 5,5 | 5,5 | 5,5 | 5,5 | 5,5 |
| **Viskosität (Brookfield, Spindel 6, 20U/min, 20°C, 1 min. (mPas)** | 18,000 | 21,000 | 16,000 | 15,500 | 22,000 |
| **Viskosität (Brookfield, Spindel 6, 20U/min, 20°C, 1 min. (mPas) nach drei Gefrier-Tau Zyklen** | 14800 | 22100 | 15800 | 12600 | 18400 |

Die Ergebnisse in der Tabelle zeigen, dass die Viskositätswerte aller erfindungsgemäßen Zusammensetzungen nach drei Gefrier-Tau-Zyklen stabil bleiben (>80% der Anfangsviskosität).

## Patentansprüche

1. Strukturierte wässrige Zusammensetzung, enthaltend ein Tensidgemisch, das - bezogen auf das Gesamtgewicht der Zusammensetzung -
(a) 3 bis 20 Gew.-% mindestens eines anionischen Tensids, das ein lineares oder verzweigtes Alkylsulfat und/oder -sulfonat ist, welches alkoxyliert sein kann,
(b) 0,5 bis 10 Gew.-% mindestens eines amphoteren/zwitterionischen Tensids und
(c) 0,5 bis 10 Gew.-% mindestens eines Alkanolamids,
welches eine lineare, verzweigte, gesättigte oder ungesättigte aliphatische C-Kette und die folgende Struktur aufweist, 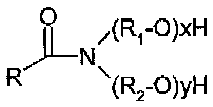 in der R eine C₈-C₂₄ gesättigte oder ungesättigte, lineare oder vernetzte aliphatische Gruppe, R₁ und R₂ gleich oder verschieden sind und eine C₂-C₄ lineare oder verzweigte aliphatische Gruppe bilden, x einen Wert von 0 bis 10 und y einen Wert von 1 bis 10 aufweist, wobei die Summe aus x+y kleiner oder gleich 10 ist, und
(d) einen Ölkörper mit einem Tropfpunkt >45°C, ausgewählt aus Petrolatum, einem Paraffin oder einem Silikon,
**dadurch gekennzeichnet, dass** die Zusammensetzung weiterhin
(e) mindestens ein kationisches Tensid (e) der Formel 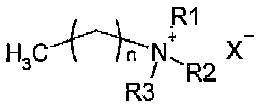 enthält, in der R1, R2 und R3 unabhängig voneinander ausgewählt sind aus - CH₃, -CH₂CH₃, -CH₂CH₂CH₃, -CH(CH₃)₂, n für einen ganzzahligen Wert von 9 bis 41 und X für ein Anion steht, und
dass die Zusammensetzung nach mindestens einem Gefrier-Tau-Zyklus eine Viskosität aufweist, die mindestens noch 80% der Anfangsviskosität beträgt.

2. Zusammensetzung nach Anspruch 1, **dadurch gekennzeichnet, dass** sie nach mindestens 3 Gefrier-Tau-Zyklen noch eine Viskosität von mindestens 80% der Anfangsviskosität aufweist.

3. Zusammensetzung nach einem der Ansprüche 1 oder 2, **dadurch gekennzeichnet, dass** als amphoteres Tensid mindestens ein amphoteres Tensid aus den Gruppen der N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren mit jeweils etwa 8 bis 24 C-Atomen in der Alkylgruppe, Alkylaminoessigsäuren mit jeweils etwa 8 bis 24 C-Atomen in der Alkylgruppe, N-Kokosalkylaminopropionat, Kokosacylaminoethylaminopropionat, C₁₂ - C₁₈ - Acylsarcosin, N-Alkyl-N,N-dimethylammonium-glycinate, beispielsweise Kokosalkyl-dimethylammoniumglycinat, N-Acyl-aminopropyl-N,N-dimethylammoniumglycinate, beispielsweise Kokosacylaminopropyldimethylammoniumglycinat, 2-Alkyl-3-carboxymethyl-3-hydroxyethyl-imidazoline mit jeweils 8 bis 18 C-Atomen in der Alkyl- oder Acylgruppe, Kokosacylaminoethylhydroxyethylcarboxymethylglycinat, der unter der INCI-Bezeichnung Cocamidopropyl Betain bekannten Verbindungen, der unter der INCI-Bezeichnung Disodium Cocoamphodiacetate bekannten Verbindungen enthalten ist.

4. Zusammensetzung nach einem der Ansprüche 1 bis 3, **dadurch gekennzeichnet, dass** sie weiterhin mindestens einen Elektrolyten enthält.

5. Zusammensetzung nach Anspruch 1, **dadurch gekennzeichnet, dass** die Reste R1, R2 und R3 identisch sind und für den Rest -CH₃ stehen, dass n für Werte von 9 bis 31, vorzugsweise von 11 bis 29, besonders bevorzugt von 13 bis 27, weiter bevorzugt von 15 bis 27 und insbesondere für 15, 16, 17, 18, 19, 20 oder 21, und dass X⁻ für ein Halogenidanion, insbesondere für Chlorid, steht.

6. Zusammensetzung nach einem der Ansprüche 1 bis 5, **dadurch gekennzeichnet, dass** sie weiterhin mindestens ein "benefit agent" enthält, das ausgewählt sein kann aus Antischuppenmitteln, Konservierungsmitteln, UV-Filtern, pflanzlichen Ölen, Verdickungsmitteln, Polymeren, Feuchthaltemitteln, Vitaminen- und/oder Provitaminen, Haarstrukturantien, Haarpflegemitteln, Haarwuchsmitteln und Mitteln gegen Haarausfall.

7. Zusammensetzung nach Anspruch 6, **dadurch gekennzeichnet, dass** sie mindestens einen Bestandteil aus der Gruppe der Proteinhydrolysate, pflanzlichen Öle, Vitamine, Aminosäuren und/oder deren Derivaten, Pflanzenextrakte, kationischen Polymere und der Haarstrukturantien enthält.

8. Kosmetische Verwendung einer Zusammensetzung nach einem der Ansprüche 1 bis 7 zur Reinigung und Pflege menschlicher Haut und menschlicher Haare.

## Claims

1. A structured aqueous composition containing a surfactant mixture which, relative to the total weight of the composition, contains
(a) 3 to 20 wt.% of at least one anionic surfactant which is a linear or branched alkyl sulfate and/or a linear or branched alkyl sulfonate which may be alkoxylated,
(b) 0.5 to 10 wt.% of at least one amphoteric/zwitterionic surfactant and
(c) 0.5 to 10 wt.% of at least one alkanolamine,
which has a linear, branched, saturated or unsaturated aliphatic C chain and the following structure, 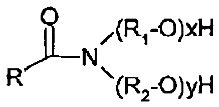 in which R is a C₈-C₂₄ saturated or unsaturated, linear or crosslinked aliphatic group, R₁ and R₂ are identical or different and form a C₂-C₄ linear or branched aliphatic group, x has a value of 0 to 10 and y a value of 1 to 10, wherein the sum of x+y is less than or equal to 10, and
(d) an oil body with a dropping point of >45°C, selected from
petrolatum, a paraffin or a silicone,
**characterised in that** the composition furthermore contains
(e) at least one cationic surfactant (e) of formula 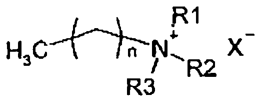 in which R1, R2 and R3 are mutually independently selected from among -CH₃, -CH₂CH₃, -CH₂CH₂CH₃, - CH(CH₃)₂, n denotes an integral value from 9 to 41 and X⁻ denotes an anion, and
**in that**, after at least one freeze-thaw cycle, the composition has a viscosity which amounts to at least 80% of the initial viscosity.

2. A composition according to claim 1, **characterised in that**, after at least 3 freeze-thaw cycles, it still has a viscosity of at least 80% of the initial viscosity.

3. A composition according to one of claim 1 or claim 2, **characterised in that** it contains as amphoteric surfactant at least one amphoteric surfactant from the groups of N-alkylglycines, N-alkylpropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids having in each case approx. 8 to 24 C atoms in the alkyl group, alkylaminoacetic acids having in each case approx. 8 to 24 C atoms in the alkyl group, N-cocoalkyl aminopropionate, cocoacylaminoethyl aminopropionate, C₁₂-C₁₈ acyl sarcosine, N-alkyl-N,N-dimethylammonium glycinates, for example cocoalkyldimethylammonium glycinate, N-acylaminopropyl-N,N-dimethylammonium glycinates, for example cocoalkylaminopropyldimethylammonium glycinate, 2-alkyl-3-carboxymethyl-3-hydroxyethylimidazolines having in each case 8 to 18 C atoms in the alkyl or acyl group, cocoacylaminoethylhydroxyethylcarboxymethyl glycinate, the compounds known by the INCI name Cocamidopropyl Betaine or the compounds known by the INCI name Disodium Cocoamphodiacetate.

4. A composition according to one of claims 1 to 3, **characterised in that** it furthermore contains at least one electrolyte.

5. A composition according to claim 1, **characterised in that** the residues R1, R2 and R3 are identical and denote the residue -CH₃, **in that** n denotes values of 9 to 31, preferably of 11 to 29, more preferably of 13 to 27, further preferably of 15 to 27 and in particular of 15, 16, 17, 18, 19, 20 or 21, and **in that** X⁻ denotes a halide anion, in particular chloride.

6. A composition according to one of claims 1 to 5, **characterised in that** it furthermore contains at least one "benefit agent" which may be selected from antidandruff agents, preservatives, UV filters, plant oils, thickeners, polymers, humectants, vitamins and/or provitamins, hair structuring agents, hair care agents, hair restorers and agents for combating hair loss.

7. A composition according to claim 6, **characterised in that** it contains at least one component from the group of protein hydrolysates, plant oils, vitamins, amino acids and/or the derivatives thereof, plant extracts, cationic polymers and hair structuring agents.

8. Cosmetic use of a composition according to one of claims 1 to 7 for cleaning and caring for human skin and human hair.

## Revendications

1. Composition aqueuse structurée, contenant un mélange d'agents tensioactifs, qui contient - par rapport au poids total de la composition -
(a) 3 à 20% en poids d'au moins un agent tensioactif anionique, qui est un alkylsulfate et/ou un alkylsulfonate linéaire ou ramifié, qui peut être alcoxylé,
(b) 0,5 à 10% en poids d'au moins un agent tensioactif amphotère/zwittérionique et
(c) 0,5 à 10% en poids d'au moins un alcanolamide,
qui présente une chaîne carbonée aliphatique linéaire, ramifiée, saturée ou insaturée et la structure suivante : 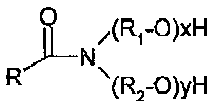 dans laquelle R représente un groupe aliphatique en C₈-C₂₄ saturé ou insaturé, linéaire ou ramifié, R₁ et R₂ sont identiques ou différents et forment un groupe aliphatique en C₂-C₄ linéaire ou ramifié, x présente une valeur de 0 à 10 et y une valeur de 1 à 10, la somme de x + y étant inférieure ou égale à 10, et
(d) un corps huileux présentant un point de goutte > 45°C, choisi parmi le pétrolatum, une paraffine ou un silicone,
**caractérisée en ce que** la composition contient en outre
(e) au moins un agent tensioactif cationique (e) de formule 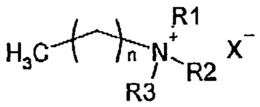 dans laquelle R1, R2 et R3 sont choisis, indépendamment les uns des autres, parmi -CH₃, -CH₂CH₃, -CH₂CH₂CH₃, -CH(CH₃)₂, n présente une valeur entière de 9 à 41 et X représente un anion, et **en ce que** la composition, après au moins un cycle de congélation-décongélation, présente une viscosité qui représente au moins encore 80% de la viscosité de départ.

2. Composition selon la revendication 1, **caractérisée en ce qu'**elle présente, après au moins 3 cycles de congélation-décongélation, encore une viscosité d'au moins 80% de la viscosité de départ.

3. Composition selon l'une quelconque des revendications 1 ou 2, **caractérisée en ce qu'**elle contient, comme agent tensioactif amphotère, au moins un agent tensioactif amphotère des groupes formés par les N-alkylglycines, les acides N-alkylpropioniques, les acides N-alkylaminobutyriques, les acides N-alkyliminodipropioniques, les N-hydroxyéthyl-N-alkylamidopropylglycines, les N-alkyltaurines, les N-alkylsarcosines, les acides 2-alkylaminopropioniques comprenant à chaque fois environ 8 à 24 atomes de carbone dans le groupe alkyle, les acides alkylaminoacétiques comprenant à chaque fois environ 8 à 24 atomes de carbone dans le groupe alkyle, le N-cocoalkylaminopropionate, le coco-acylaminoéthylaminopropionate, la C₁₂-C₁₈-acylsarcosine, les glycinates de N-alkyl-N,N-diméthylammonium, par exemple le glycinate de coco-alkyldiméthylammonium, les glycinates de N-acylaminopropyl-N,N-diméthylammonium, par exemple le glycinate de coco-acylaminopropyldiméthylammonium, les 2-alkyl-3-carboxyméthyl-3-hydroxyéthylimidazolines comprenant à chaque fois 8 à 18 atomes de carbone dans le groupe alkyle ou acyle, le cocoacylaminoéthylhydroxyéthylcarboxyméthylglycinate, les composés connus sous la dénomination INCI "Cocamidopropyl Betain", les composés connus sous la dénomination INCI "Disodium Cocoamphodiacetate".

4. Composition selon l'une quelconque des revendications 1 à 3, **caractérisée en ce qu'**elle contient en outre au moins un électrolyte.

5. Composition selon la revendication 1, **caractérisée en ce que** les résidus R1, R2 et R3 sont identiques et représentent le résidu -CH₃, n présente des valeurs de 9 à 31, de préférence de 11 à 29, de manière particulièrement préférée de 13 à 27, plus préférablement de 15 à 27 et en particulier de 15, 16, 17, 18, 19, 20 ou 21, et **en ce que** X⁻ représente un anion d'halogénure, en particulier le chlorure.

6. Composition selon l'une quelconque des revendications 1 à 5, **caractérisée en ce qu'**elle contient en outre au moins un "benefit agent" (agent bénéfique), qui peut être choisi parmi les agents antipelliculaires, les conservateurs, les filtres UV, les huiles végétales, les épaississants, les polymères, les humectants, les vitamines et/ou les provitamines, les structurants des cheveux, les agents de soins des cheveux, les agents de croissance pour cheveux et les agents contre la perte des cheveux.

7. Composition selon la revendication 6, **caractérisée en ce qu'**elle contient au moins un constituant du groupe des hydrolysats de protéines, des huiles végétales, des vitamines, des acides aminés et/ou leurs dérivés, des extraits végétaux, des polymères cationiques et des structurants des cheveux.

8. Utilisation cosmétique d'une composition selon l'une quelconque des revendications 1 à 7 pour le nettoyage et le soin de la peau humaine et des cheveux humains.
